# EUROPEAN PATENT SPECIFICATION

(11) **EP 2 006 302 B1**
(45) Date of publication and mention of the grant of the patent: **12.03.2014**
(21) Application number: 07741817.6
(22) Date of filing: 11.04.2007
(51) Int. Cl.: C07K 1/107, C07K 14/47, A61K 39/00, A61K 39/39, A61P 35/00

(54) **PROTEIN COMPLEX AND PROCESS FOR PRODUCTION THEREOF**
PROTEINKOMPLEX UND VERFAHREN ZU DESSEN HERSTELLUNG
COMPLEXE PROTEINIQUE ET PROCEDE DE PRODUCTION DUDIT COMPLEXE

(30) Priority: 11.04.2006 JP 2006108668
(43) Date of publication of application: 24.12.2008
(73) Proprietor: ImmunoFrontier, Inc., Ota-ku, Tokyo 143-0023 (JP)
(72) Inventor: NAGURA, Kenji, Koganei-shi, Tokyo 184-0014 (JP); HARADA, Naozumi, Tokyo 103-0023 (JP); URUSHIHARA, Masahiro, Yokohama-shi, Kanagawa 221-0863 (JP)
(74) Representative: Jappy, John William Graham
(86) International application number: PCT/JP2007/058381
(87) International publication number: WO 2007/119859

(56) References cited:
- WO-A1-87/01729
- WO-A1-98/09650
- WO-A2-99/40188
- MUKHOPADHYAY A: "Reversible protection of disulfide bonds followed by oxidative folding render recombinant hCGbeta highly immunogenic", VACCINE, ELSEVIER LTD, GB, vol. 18, no. 17, 1 March 2000 (2000-03-01) , pages 1802-1810, XP004190060, ISSN: 0264-410X, DOI: DOI:10.1016/S0264-410X(99)00482-X
- MARASKOVSKY EUGENE ET AL: "NY-ESO-1 protein formulated in ISCOMATRIX adjuvant is a potent anticancer vaccine inducing both humoral and CD8+ t-cell-mediated immunity and protection against NY-ESO-1+ tumors.", CLINICAL CANCER RESEARCH : AN OFFICIAL JOURNAL OF THE AMERICAN ASSOCIATION FOR CANCER RESEARCH 15 APR 2004 LNKD- PUBMED:15102697, vol. 10, no. 8, 15 April 2004 (2004-04-15) , pages 2879-2890, XP002615263, ISSN: 1078-0432
- CLARK E D B: "Protein refolding for industrial processes", CURRENT OPINION IN BIOTECHNOLOGY, LONDON, GB, vol. 12, no. 2, 1 April 2001 (2001-04-01), pages 202-207, XP002496443, ISSN: 0958-1669, DOI: DOI:10.1016/S0958-1669(00)00200-7
- CHAN W W-C: "A METHOD FOR THE COMPLETE S SULFONATION OF CYSTEINE RESIDUES IN PROTEINS", BIOCHEMISTRY, AMERICAN CHEMICAL SOCIETY, US, vol. 7, no. 12, 1 December 1968 (1968-12-01), pages 4247-4254, XP001022551, ISSN: 0006-2960, DOI: DOI:10.1021/BI00852A016
- HEATH W.F. ET AL.: '(A-C-B) Human Proinsulin, a Novel Insulin Agonist and Intermediate in the Synthesis of Biosynthetic Human Insulin' THE JOURNAL OF BIOLOGICAL CHEMISTRY vol. 267, no. 1, 1992, pages 419 - 425, XP003018538
- SHIBATA R. ET AL.: 'INDUCTION OF IN VITRO AND IN VIVO ANTI-TUMOR RESPONSES BY SENSITIZATION OF MICE WITH LIPOSOMES CONTAINING A CRUDE BUTANOL EXTRACT OF LEUKEMIA CELLS AND TRANSFERRED INTER-MEMBRANOUSLY WITH CELL-SURFACE PROTEINS' INT. J. CANCER vol. 48, no. 3, 1991, pages 434 - 442, XP003018539
- DAVIS I.D. ET AL.: 'Recombinant NY-ESO-1 protein with ISCOMATRIX adjuvant induces broad integrated antibody and CD4+ and CD8+ T cell responses in humans' PNAS vol. 101, no. 29, 20 July 2004, pages 10697 - 10702, XP002451138

## Description

### TECHNICAL FIELD

The present invention relates to a protein complex and a process for producing the same. More specifically, the present invention relates to a protein antigen of an anti-cancer protein vaccine and a process for producing the same. The present invention may most preferably be applied to the cancer-specific protein antigen of an anti-cancer protein vaccine having two or more cysteine residues in the molecule, and a process for producing the same. In one aspect, the present invention relates to protecting the cancer-specific protein antigen of an anti-cancer protein vaccine by oxidative sulfonation of cysteine residues of the vaccine having two or more cysteine residues in the molecule like the cancer-testis antigen (CTA) in order to efficiently purify the antigen.

### BACKGROUND ART

Though the present invention can be applied without limitation to a protein having two or more cysteine residues in the molecule, the background technology related to the above cancer-testis antigen (CTA) is explained first for the sake of convenience.

It was known that the cytotoxic T lymphocytes (CTL) of a patient appear to recognize and attack an antigen specifically expressed in cancer cells, and from a melanoma cell line, the gene sequence of the MAGE-A1 antigen was identified for the first time by the gene transfection approach (Non-patent document 1: van der Bruggen P. et al., Science 254: 1643-7 (1991)). By hybridizing a cosmid containing a probe derived from this MAGE-A1 gene with a DNA library, a MAGE family comprising eleven closely-related members was newly identified (Non-patent document 2: De Plaen E. et al., Immunogenetics 40: 360-9 (1994)). These 12 MAGE-A genes were localized at the q28 region of the X chromosome (Non-patent document 3: Rogner UC et al., Genomics 29: 725-31 (1995)).

Thereafter, the cancer-testis antigen (CTA) was continuously discovered by the SEREX method (see, for example, Sahin, U. et al., (1995) Proc. Natl. Acad. Sci. U.S.A. 92: 11810-3; Chen YT et al., (1997) Proc. Natl. Acad. Sci. U.S.A. 94: 1914-8; Tureci O. et al., (1996) Cancer Res. 56: 4766-72). It was demonstrated that most of these CTA's are only present in the testicular cells for the normal cells, and are frequently expressed in cancer cells. Because of these characteristics, CTA is considered to be an ideal target antigen for cancer vaccine. In fact, a multitude of attempts have been made so far to develop peptide vaccines using as the antigen an epitope (an oligopeptide comprising 8-10 amino acid residues) through which CTA recognizes the CTA-positive cell.

However, the activation of CTL alone is not sufficient for a vaccine effect in many cases, and furthermore since the recognition epitopes differ with the type of human leukocyte antigen (HLA), it was thought, the clinical application of peptide vaccines is limited. In contrast, it is known that since a proteinaceous vaccine that uses the full length or partial length of CTA contains not only a CTL-recognizing epitope but a helper T cell-recognizing epitope, it can activate various types of T cells at the same time, and that it can function as a so-called multivalent vaccine that contains a plurality of recognizing epitopes for different HLA.

A proteinaceous vaccine may often be used by embedding a cancer specific antigen in a polymer carrier such as liposome in order to facilitate efficient delivery of the cancer specific antigen into an antigen presenting cell (for example dendritic cell) or to facilitate stabilization of a cancer specific antigen. One such polymer carrier is an aggregate of hydrophobized polysaccharides (such as CHP) obtained by adding a hydrophobic group of cholesterol etc. to a polysaccharide such as pullulan. CHP has a property of facilitating antigen presentation by the antigen presenting cell by embedding the entire cancer specific antigen, and also has an effect of enhancing stability as a pharmaceutical formulation by protecting the cancer specific antigen within CHP (Patent document 1: WO 98/09650 publication). As such a carrier, ISCOMATRIX adjuvant is known (Patent document X: WO 2005032475).

As a polymer carrier that serves as an embedding agent, a known substance such as a liposome and ISCOMATRIX are included.

Liposomes are generally used for such purposes and known to those skilled in the art, and are used in DDS systems by combining with a phospholipid and various other substances such as cholesterol or by being conferred a function of concentrating to a target site of interest using antibody or magnetic particles. See, for example, a vaccine containing a butanol-extract of leukemia cells (Shibata R. et al., (1991) Int. J. Cancer, May 30, 48(3): 434-42) or a vaccine from a membrane protein (Sunamoto J. et al., (1990) Ann. NY Acad. Sci. 613: 116-27).

ISCOMATRIX is a known substance that can be made a vaccine by embedding a cancer antigen, and is composed of saponin, cholesterol and a phospholipid. It is being developed by Australia's CSL Limited and there are already reports on clinical studies (see, for example, Butts C. et al., (2005) J. Clin. Oncol. 20; 23(27): 6674-81; North S. et al., (2005) Expert Rev. Vaccines 4(3): 249-57; Davis D. et al., (2004) P. Natur. Acad. Sci. 101 (29) 10697).

Though cancer specific antigens such as the above CTA can be produced by expression in recombinant Escherichia coli (E. coli), the production usually requires several steps of purification in order to secure the quality of grade commensurate with pharmaceutical drugs. In many cases, a tag called His-Tag, a sequence in which a plurality of histidine are arranged and which forms a chelate with a metal ion, is added to the N-terminal or the C-terminal of a cancer-specific protein, followed by the affinity purification of the cancer-specific protein (see Skerra A. et al., Biotechnology (1991) Mar., 9(3): 273-8, and Crowe J. et al., Methods Mol. Biol. (1994) 31: 371-387). However, cancer-specific antigens such as CTA often have a plurality of thiol (-SH) groups of cysteine, and tend to form multimers by disulfide bonding (-S-S-) between protein molecules.

By a refolding procedure, some proteins can be intramolecularly crosslinked to improve their characteristics, i.e. changed to monomers, thereby enabling the purification as pure proteins. However, cancer-specific antigens such as CTA are not easy to form intramolecular disulfide bonds, and it is difficult to obtain monomers even by the refolding procedure. Therefore, the purification of cancer-specific antigens such as CTA have the various problems as shown below:
(1) Proteins containing cysteines, when concentrated during the purification process, may be condensed due to intermolecular sulfide bonding and form multimers leading to reduced yield;
(2) Proteins containing thiol groups are generally unstable, and have a stability problem when they are intended to be used as pharmaceuticals;
(3) In the case of protein antigens for vaccine formulations, attempts to purify with thiol groups protected require the reconstitution of epitope thiol groups to be presented as antigens (i.e. they must be reversible).

In the production of NY-ESO-1, a vaccine having CTA as the antigen, non-reduced SDS cannot control the ratio of multimers, and thus reduced SDS-PAGE alone is used in quality control, and the 8 M-urea denatured product per se is used as a bulk drug (Non-patent document 4: Preparative Biochem. & Biotech. 35: 119-134, 2005). It is reported, however, this requires the removal of urea during the process of drug formulation, during which process multimer formation may resume, and therefore an ordinary protein when included in the carrier ISCOMATRIX adjuvant may become 40 nm nanoparticles, whereas NY-ESO-1 became gigantic 2000 nm aggregates (Non-patent document 5: Clin. Cancer Res. 10: 2879-2890, 2004).

In an attempt to solve the above problems, a method was devised in which after CTA termed as MAGE-A3 was reduced with 2-mercaptoethanol, iodoacetamide was acted on it to block the thiol groups by carboxymethylation (Patent document 2: WO 99/40188 publication). As a method of selectively protecting thiol groups like this, carboxymethylation with iodoacetic acid or iodoacetamide is commonly carried out. However, this method has a drawback that since it blocks thiol groups in an irreversible manner, protein epitopes may be inactivated in the case of protein epitopes containing cysteine.

Also, in the production of recombinant proteins containing cysteine with E. coli as the host, reconstitution (refolding) with a REDOX reagent (glutathione-oxidized glutathione or cysteine-cysteine etc.) is required, and in the production of some proteins, for example GM-CSF (Non-patent document 6: Behring Inst Mitt. 1988 Aug: (83): 246-9) and insulin (Non-patent document 7: J. Biol. Chem. 1992 Jan. 5, 267(1): 419-25, Non-patent document 8: Anal. Chem. 1992 Mar. 1: 64(5): 507-11), there are reported methods in which sodium sulfite and sodium tetrathionate are acted to sulfonate the sulfide (-SH) group, and, after purification, it is refolded.

Sulfonation has been used for immunoglobulins and are known to those skilled in the art (see, for example, the package insert of the freeze-dried sulfonated human immunoglobulin Kenketsu Venilon-I)
[Patent document 1] WO 98/09650 publication
[Patent document 2] WO 99/40188 publication
[Non-patent document 1] van der Bruggen P. et al., Science 254: 1643-7 (1991)
[Non-patent document 2] De Plaen E. et al., Immunogenetics 40: 360-9 (1994)
[Non-patent document 3] Rogner UC et al., Genomics 29: 725-31 (1995)
[Non-patent document 4] Preparative Biochem. & Biotech. 35: 119-134, 2005
[Non-patent document 5] Clin. Cancer Res. 10: 2879-2890, 2004
[Non-patent document 6] Behring Inst Mitt. 1988 Aug: (83): 246-9
[Non-patent document 7] J. Biol. Chem. 1992 Jan. 5, 267(1): 419-25
[Non-patent document 8] Anal. Chem. 1992 Mar. 1: 64(5): 507-11

Mukhopadhyay A. Vaccine, 18(17):1802-1810 (2000) discloses a vaccine containing recombinant human chorionic gonadotropin (hCG) produced by a process including a sulfonation step to improve antigen stability and recovery.

Clark EDB. Current Opinion in Biotechnology, 12(2):202-207 (2001) discusses methods for refolding proteins isolated from bacterial inclusion bodies including sulfonation steps to prevent oxidation of reduced cysteines.

Chan WW-C. Biochemistry, 7(12):4247-4254 (1968) discloses a method for complete sulfonation of cysteine residues in proteins under mild conditions.

WO 87/01729 A1 discloses methods useful for amidation of recombinant polypeptides containing cysteine or cystine residues preventing their aggregation and allowing recovery of proteins with improved activity.

### DISCLOSURE OF THE INVENTION

It is an object of the present invention to provide a process for producing a cancer-testis antigen-hydrophobized polysaccharide complex that has solved the above problems associated with prior art.

More specifically, it is an object of the present invention to provide a process for producing a complex of a complex-forming cancer-testis antigen that permits its purification at high yield without forming aggregates due to intermolecular disulfide bond formation between the cancer-testis antigens.

After intensive and extensive research, the present inventor has found that the above object can be attained by sulfonating the thiol groups of a cancer-testis antigen protein having two or more cysteine residues in the molecule, and purifying it with a column chromatography method etc. while keeping the monomer state, and then allowing them to form a complex with a fine particle-forming substance such as a hydrophobicity-imparted polysaccharide, and, if desired, reducing the sulfonated protein in the complex.

The process for producing a cancer-testis antigen complex of the present invention is based on the above finding, and more specifically it is a process for producing a cancer-testis antigen hydrophobized polysaccharide complex which comprises complexing a cancer-testis antigen having two or more cysteine residues in the molecule with a hydrophobized polysaccharide, wherein prior to embedding in the above hydrophobized polysaccharide, the cysteine residues of the cancer-testis antigen are modified followed by purification.

In accordance with the present invention, a cancer-specific cancer-testis antigen having two or more cysteine residues in the molecule such as CTA can be obtained as stable monomers at a high yield.

According to the present invention, it is possible to reconstitute the thiol groups of the sulfonated recombinant cancer-specific cancer-testis antigen without forming disulfide bonds between the cancer-testis antigen molecules by reducing before or after embedding in a polymer carrier such as a hydrophobicity-imparted polysaccharide.

According to the finding by the present inventor, it is estimated that by oxidative sulfonation of a cancer-specific cancer-testis antigen having two or more cysteine residues in the molecule, the cancer-specific cancer-testis antigen is stabilized and intermolecular bond formation between the cancer-testis antigen can be effectively prevented, thereby permitting the purification of the cancer-testis antigen monomer at high yield.

In contrast, in the conventional purification method of cancer-specific antigens such as CTA (a kind of protein antigen), according to the finding by the present inventor, many cancer-specific antigens contain odd numbers of cysteine residues, and fixed amounts of these cysteines occur as thiol groups without forming intramolecular disulfide bonds or form disulfide bonds between molecules contributing to the formation of CTA multimers, and thus the presence of these thiol groups has inhibited the efficient purification of cancer-specific antigens.

The present invention, for example, comprises the following aspects:
[1] A process for producing a cancer-testis antigen-embedding agent complex, the method comprising complexing a cancer-testis antigen having two or more cysteine residues in the molecule and a hydrophobized polysaccharide, wherein
   prior to embedding in the hydrophobized polysaccharide, the cysteine residues of the cancer-testis antigen are sulfonated, and then purified.
[2] The process for producing a cancer-testis antigen complex according to [1] wherein the embedding agent is a hydrophobicity-imparted polysaccharide.
[3] The process for producing a protein complex according to any of [1] to [2] wherein the modification is reversible.
[4] The process for producing a cancer-testis antigen complex according to any of [1] to [3] wherein the cancer-testis antigen (CTA) is a recombinant testis antigen expressed using a recombinant Escherichia coli.
[5] The process for producing a cancer-testis antigen complex according to [1] which method comprises, after the purification of the protein, the step of reducing the purified protein with a sulfide bond reducing agent prior to complex formation with the hydrophobized polysaccharide.
[6] The process for producing a cancer-testis antigen complex according to [1] which method comprises the step of reducing the complex with a sulfide bond reducing agent after the cancer-testis antigen has been complexed with the hydrophobized polysaccharide.
[7] The process for producing a cancer-testis antigen complex according to any of [1] to [3] wherein the sulfonation is carried out by allowing a thiosulfate and a tetrathionate to act on the protein to sulfonate the thiol groups of the protein in order to protect them.
[8] The process for producing a cancer-testis antigen complex according to [1] wherein the purification is carried out by chromatography.

### [EFFECT OF THE INVENTION]

According to the present invention, as described above, there is provided a cancer-specific cancer-testis antigen composition and a process for producing the same that permit the preferred purification of the cancer-testis antigen while suppressing as much as possible the polymerized multimer formation of the cancer-specific cancer-testis antigen.

### BRIEF EXPLANATION OF THE DRAWINGS

Fig. 1 shows that the reduced SDS-PAGE of the cell disrupted liquid obtained by culturing MAGE-A4-recombinant Escherichia coli demonstrates the presence of MAGE-A4 in the supernatant of the disrupted liquid.
Fig. 2 shows that the reduced SDS-PAGE of the cell disrupted liquid of the His-Tag-MAGE-A4-expressed Escherichia coli and its crude fraction with the Ni-affinity column demonstrates that one time purification yields MAGE-A4 with a purity of 95% or more.
Fig. 3 is the nonreduced SDS-PAGE of a crude-purified His-Tag-MAGE-A4 and its sulfonation-protected product. It shows that the crude purified MAGE-A4 prior to the protection reaction is an assembly of multimers (6-110 kd) and structural isomers (38-48 kd) in the nonreduced state, and that they converge to one band (49 kd) by the sulfonation protection reaction.
Fig. 4 is the nonreduced SDS-PAGE of a crude-purified His-Tag-MAGE-A4 and its sulfonation-protected product treated under a harsh condition. Fig. 4 shows that the products prior to protection become polymers and aggregate, whereas the sulfonation-protected product is stable to heat or the freeze-thaw treatment.

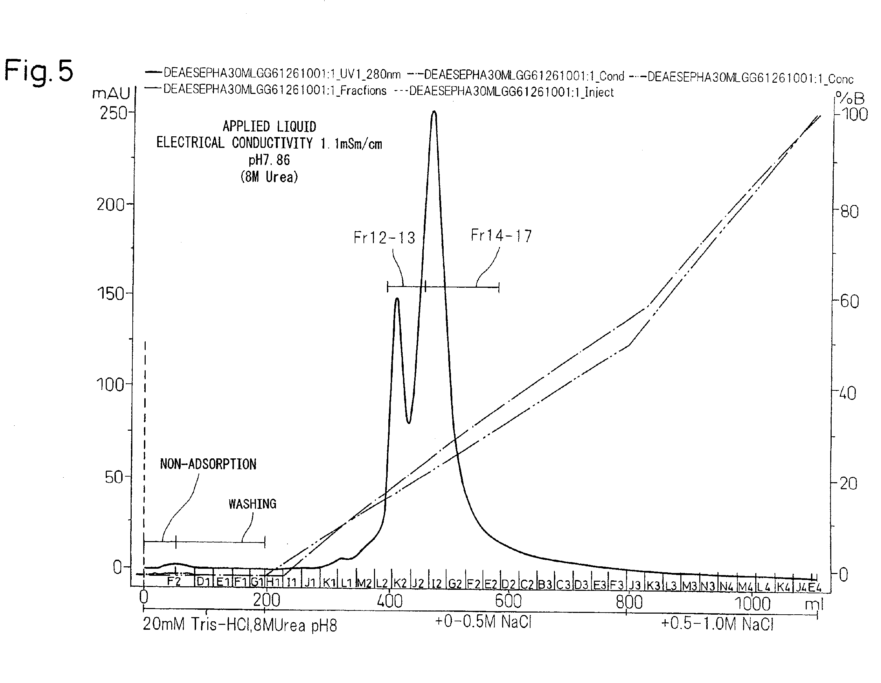

Fig. 5 is a purification chart with a DEAE column. In the chart two elution peaks were observed, but they could not be distinguished from SDS-PAGE or RPC, and were estimated to be due to differences in conformation.
Fig. 6 is a chart showing the result of ELISPOT assay of CHP-His-Tag-MAGE-A4. Significantly more INF-γ spots are recognized compared to the negative control (non-treat, CHP-Her2), confirming that CHP-MAGE has been incorporated into DC and activates CTL.
Fig. 7 is a chart showing the result of ELISPOT assay of CHP-MAGE-A4. Significantly more INF-γ spots are recognized compared to the negative control (non-treat, CHP-Her2), confirming that CHP-His-Tag-MAGE-A4 has been incorporated into the dendritic cells and activates CTL.
Fig. 8 is a graph showing the size distribution (based on the intensity of a given peak) of the embedded product shown in Fig. 2.

### BEST MODE FOR CARRYING OUT THE INVENTION

The present invention will now be explained in further detail with reference to drawings as needed. As used hereinbelow, "part" and "%" that represent amount ratio are based on weight unless otherwise specified.

### (Cancer-bearing protein vaccine)

The cancer-bearing cancer-testis antigen vaccine of the present invention may be formulated by embedding a cancer-specific cancer-testis antigen having two or more cysteine residues in the molecule in a polymer carrier such as a hydrophobicity-imparted polysaccharide.

### (Reconstitution of thiol groups)

Though, as used herein, the cancer-testis antigen may be embedded in a hydrophobicity-imparted polysaccharide etc. as it is sulfonated, it may be reduced immediately before being embedded in the hydrophobicity-imparted polysaccharide, or after being embedded in the hydrophobicity-imparted polysaccharide. This reduction method may be realized by acting 2 mM or more of 2-mercaptoethanol, dithiothreitol, cysteine etc.

### (Polymer carrier)

The hydrophobicity-imparted polysaccharide (used in the sense that "hydrophobicity-imparted polysaccharide-assembled fine particles" are included; the same hereinafter) may be produced by a method known per se as described, for example, Akiyoshi et al., Macromolecules, 6, pp. 3062-3068, 1993; Akiyoshi et al., J. Proc. Japan. Acad., 71, 71B, p. 15, 1995; JP-A (Japanese Unexamined Patent Publication; Kokai) No. 61-69801, JP-A No. 3-292301, JP-A No. 7-97333.

The polysaccharide in the hydrophobicity-imparted polysaccharide is not specifically limited as long as the sugar residue is a glycoside-linked polymer. As the sugar residue that constitutes the polysaccharide, residues derived from a monosaccharide such as glucose, mannose, galactose and fucose, a disaccharide, or an oligosaccharide can be used. The sugar residue may 1,2-, 1,3-, 1,4- or 1,6-glycoside linked, and the bond may be any of the α-form or the β-form. Also, the polysaccharide may be a linear or branched chain. As the sugar residue, the glucose residue is preferred, and as the polysaccharide, natural or synthetic pullulan, dextran, amylose, amylopectin, or mannan may be used, with mannan or pullulan being preferred.

The hydrophobic residue is not specifically limited, and depending on the molecular weight or isoelectric point of the antigen molecule to be embedded (or encapsulated), the one having a high rate of encapsulation may be selected. From the viewpoint of safety as a vaccine formulation, the hydrophobic group is preferably 1-5 (5% or less in terms of weight ratio) single stranded or double stranded alkyl groups or sterol residues introduced per 100 monosaccharides.

As the sterol residue, there can be mentioned the cholesterol, the stigmasterol, β-sitosterol, lanosterol, ergosterol residues, etc., and from the viewpoint of safety as a vaccine formulation, the cholesterol residue may preferably be used. As the alkyl group, the one having 20 or less carbons, more preferably 10-18 carbons, can be used, and it may be either a linear or branched chain.

As one aspect of preferred hydrophobicity-imparted polysaccharide, for example, there can be mentioned a hydrophobicity-imparted polysaccharide in which the primary hydroxy group of 1-5 sugar units per 100 sugar units constituting the polysaccharide is represented by the following formula (I):

-O-(CH₂)ₘCONH(CH₂)ₙNY-CO-O-R (I)

wherein R represents an alkyl group or a sterol residue; m represent 0 or 1; and n represent any positive integer. As used herein, as the alkyl group or the sterol residue, those mentioned above may be used, and n is preferably 1-8. Also, the hydrophobicity-imparted polysaccharide may be one bound via a linker as described in JP-A No. 7-97333.

### (Specific examples of preferred polymer carriers)

More specifically, a hydrophobicity-imparted polysaccharide (A) described below may preferably be used in the present invention.

### (1) CHP (cholesterol-hydrophobized pullulan), (2) CHM (cholesterolhydrophobized mannan), and (3) ISCOMATRIX.

As the above CHP, CHM etc., a commercially available product (for example, a commercially available product from NOF Corp. with a molecular weight of 60,000, 100,000 etc.: There is about 1.6 cholesterol per 100 sugar residues) can be used as needed.

### (Protein antigen)

As used herein, as the cancer-specific cancer-testis antigen to be embedded in the above hydrophobicity-imparted polysaccharide polymer carrier, a cancer-testis antigen that is specifically expressed in a cancer cell having two or more cysteine residues in the molecule may be used.

### (Preferred antigen)

As used herein, as the above cancer-specific cancer-testis antigen, for example the following can be preferably used.
(1) Cancer-testis antigen (CTA);
(2) recombinant cancer-testis antigen expressed using a recombinant Escherichia coli.

For example, one or a plurality of a cancer-testis antigen selected from the MAGE family of MAGE-A1, MAGE-A2, MAGE-A3, MAGE-A4, MAGE-A5, MAGE-A6, MAGE-A7, MAGE-A8, MAGE-A9, MAGE-A10, MAGE-A11, MAGE-A12, MAGE-B1, MAGE-B2, MAGE-B3, MAGE-B4, MAGE-C1, and MAGE-C2;

One or a plurality of a cancer-testis antigen selected from NY-ESO-1, the LAGE family, the GAGE family, the SAGE family, and the XAGE family;

### (3) His-Tag-added His-Tag-cancer-specific antigen in which His-Tag was added to the above (1) or (2).

### (A method of embedding an antigen by a hydrophobicity-imparted polysaccharide)

A complex of a hydrophobicity-imparted polysaccharide and an antigen may be produced by mixing the hydrophobicity-imparted polysaccharide and the antigen in the presence of a denaturant such as urea at room temperature, and then removing the denaturant by gel filtration or gel chromatography (Nishikawa, Macromolecules, 27, pp. 7654-7659, 1994). Alternatively, it may be produced by merely mixing the hydrophobicity-imparted polysaccharide and the antigen at 50-60°C even in the absence of a denaturant. The complex of the hydrophobicity-imparted polysaccharide and the antigen thus produced may be used per se as the vaccine formulation of the present invention, and, as needed, it may be subjected to a treatment such as filtersterilization.

### (Immunization with a complex of a hydrophobicity-imparted polysaccharide and an antigen)

The virus formulation that may be produced according to the present invention may be administered, as other known vaccine formulations are administered, at a given amount to an animal to immunize the animal, and similarly the titer of the vaccine formulation may be evaluated. The administration pathway of the vaccine formulation of the present invention may generally be subcutaneous or intradermal injection. The dosage of the vaccine formulation of the present invention required for immunization may be determined as appropriate, and the normal dosage as the antigen, for example, may be about 50-1500 µg/dose, preferably 100-600 µg/dose, and the number of administration may be suitably 3-30 times. Combined use of an immunopotentiating agent such as GM-CSF, IL-2, CpG and OK-432 may also be effective.

When the vaccine formulation of the present invention is formulated, a dosage form known per se, a carrier or a diluent generally used may be used as appropriate depending on the administration pathway used.

### (Production of the recombinant antigen)

The preparation of a recombinant Escherichia coli may be attained by integrating the DNA of CTA into a common plasmid. The DNA sequence of CTA is known (U.S. Pat. No. 5342774), and may easily be carried out by a person skilled in the field of biotechnology.

### (Sulfonation of the antigen)

As an example of a method of modifying the cysteine residue, sulfonation may be explained.

As used herein, when a cancer-specific protein antigen having two or more cysteine residues in the molecule is sulfonated, for example, the sulfonation condition may preferably be 30-3000 mM (preferably 200-500 mM) of sodium sulfite and 6-600 mM (preferably 30-100 mM) of tetrathionic acid reacted at 10-50°C reacted in the presence of 8M urea or 6M guanidine.

### (Method of confirming the purity of the sulfonated protein antigen)

The purity of the sulfonated protein antigen may be checked by the reversed phase HPLC or electrophoresis. The stability may be confirmed in that even the heating of it in a neutral buffer at 60°C for 10 minutes may yield one band in electrophoresis. When it is not protected by sulfonation, high molecular weight bands thought to be polymers or multimers may become increased (see the corresponding electrophoresis pattern in the Working Examples below).

In addition to sulfonation, there can also be used a method of reversibly modifying the -SH group of cysteine such as glutathionation.

### (Method of embedding in the hydrophobicity-imparted polysaccharide)

As an example of embedding, a case of using CHP may be explained.

The method of embedding in CHP may be one previously known per se (for example, WO 98/09650 publication). The outline of this embedding method is: CHP is dissolved in a denaturant solution such as 6-8 M urea or 6 M guanidine, or CHP is dissolved in water or a buffer solution and mixed at a weight ratio of 10-20 parts of CHP relative to one part of the protein, and then changed to a buffer for drug preparation by a method of ultra filtration, gel filtration, etc. and, as needed, filter-sterilized. The dissolution of CHP may sufficiently be carried out at room temperature and may also be carried out at a heated condition. The molecular weight of CHP may preferably be 50-100 kd. Also, CHP is commercially available from NOF Corp.

With regard to the above references, complex formation with liposome or ISCOMATRIX may easily be carried out by a person skilled in the art.

### (Measurement of particle size distribution of the complex)

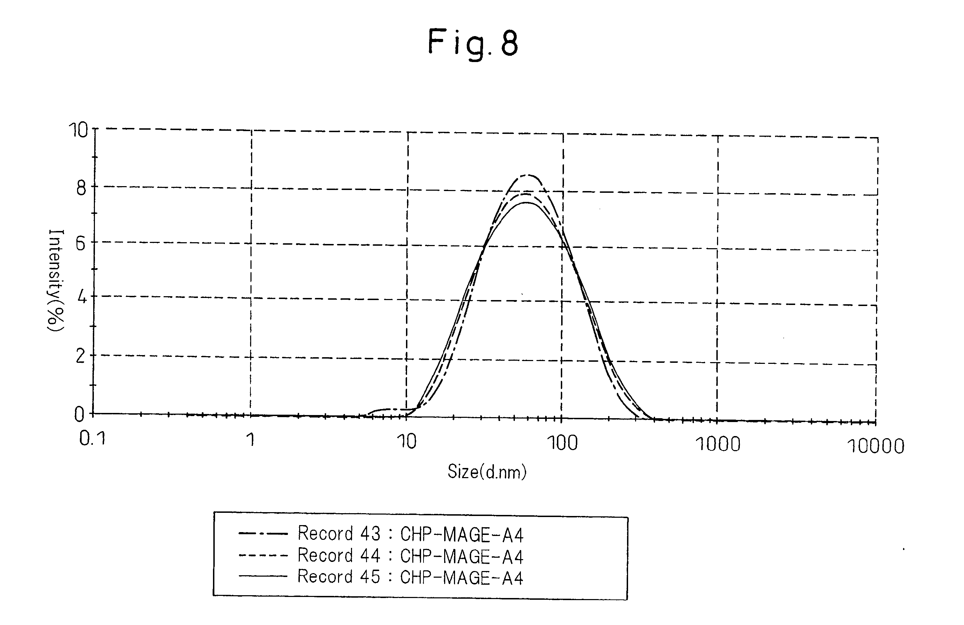

The particle size distribution of the complex of the present invention of the hydrophobized polysaccharide (A) and the protein antigen (B) may be confirmed using a granulometer (DRI) (see Fig. 8 in Working Example).

### (Confirmation of being embedded)

Embedding can be checked by GPC (gel permeation chromatography; see, for example, the description "CHP embedding" described below). This "embedding" may be confirmed by the complete transition of respective peaks of CHP and the cancer-specific antigen protein to the in the of the CHP peak in the GPC chart. As used herein, the yield of this "embedding" may preferably be 95% or more, and more preferably 99% or more.

### (Combined screening of preferred "cholesteryl polysaccharide-antigen molecule")

As used herein, the confirmation method (GPC) as described above may be used in a screening test for searching the combination of "cholesteryl polysaccharide-antigen molecule". In such screening, the yield of "embedding" may be determined. In accordance with the present invention, the yield of this "embedding" may preferably be 95% or more, and more preferably 99% or more.

### (Antigen activity after embedding)

Various methods may be available for testing the antigen activity after embedding, and for example it may be measured in an in vivo experimental system that combines the antigen presenting cells and the killer or helper T cell. Thus, an embedded protein antigen may, for example, be incorporated into an antigen presenting cell such as a dendritic cell, and the activation of a killer T cell by the antigen presenting cell may be confirmed by the ELISA method (enzyme-linked immunosorbent assay) or the ELISPOT method (enzyme-linked immunoSPOT) which measures interferon γ as the target. In such an experimental system, antigen activity may be observed.

### (Vaccine)

The embedded protein antigen of the present invention may be used as a vaccine. Since this vaccine is a so-called "antigen drug", it exhibits an immunization effect at an amount one order less than that of the so-called "antibody drug".

### (Aspect of a preferred vaccine)

As used herein, a preferred embodiment of a vaccine is as follows:
(1) A CHP-sulfonated antigen vaccine having a sulfonated recombinant testis antigen embedded in a hydrophobized polysaccharide, which is obtained by purifying a sulfonated recombinant cancer-specific protein antigen (hereinafter referred to as the "sulfonated antigen") obtained by reacting a sulfite and a tetrathionate to a recombinant cancer-specific protein antigen (hereinafter referred to as the "SH antigen") expressed using a recombinant Escherichia coli, and then by embedding the sulfonated recombinant testis antigen in a hydrophobized polysaccharide.
(2) A CHP-SH antigen vaccine which is a reduced sulfonated recombinant testis antigen of the above (I).
(3) A CHP-CTA sulfonated antigen vaccine or its reduced CHP-CTA sulfonated antigen vaccine, wherein the recombinant cancer-specific protein antigen of the above (1) or (2) is one or a plurality of CTA's selected from the MAGE family of MAGE-A1, MAGE-A2, MAGE-A3, MAGE-A4, MAGE-A5, MAGE-A6, MAGE-A7, MAGE-A8, MAGE-A9, MAGE-A10, MAGE-A11, MAGE-A12, MAGE-B1, MAGE-B2, MAGE-B3, MAGE-B4, MAGE-C1, and MAGE-C2.
(4) A CHP-CTA sulfonated antigen vaccine or its reduced CHP-CTA sulfonated antigen vaccine, wherein the recombinant testis antigen of the above (1) or (2) is one or a plurality of CTA's selected from NY-ESO-1, the LAGE family, the GAGE family, the SAGE family, and the XAGE family.
(5) A CHP-His Tag CTA sulfonated antigen vaccine or a CHP-His Tag CTA SH antigen vaccine, wherein the recombinant cancer-specific testis antigen of the above (1) or (2) is His-Tag-added His-Tag-CTA.

The present invention will now be explained with reference to Examples, but it should be noted that the present invention is not limited to these examples in any way.

### Examples

### Production Example 1

### (1) Integration of His-Tag-MAGE-A4 and the preparation of the seed stock

Using the following sequence: Forward primer: 5'-GGATCCATGTCTTCTGAGCAGAAGAGT-3', Reverse primer: 5'-AAGCTTCTCATGCTCAGACTCCCTC-3' as the primer, RT-PCR was performed on RNA derived from a melanoma cell line SK-MEL-37. As a result, the full-length cDNA of MAGE-A4 having a BamHI site (GGATCC) and a HindIII site (AAGCTT) on both ends was obtained (performed by Dr. Yao-Tseng Chen of Cornell University, Faculty of Medicine, USA).

This cDNA was integrated in the pQE9 vector (Qiagen) for His-Tag fusion protein expression to obtain a pQE9/MAGE-A4 plasmid (performed by Dr. Yao-Tseng Chen of Cornell University, Faculty of Medicine, USA). This was introduced into the competent cell M15 [pREP4] (Qiagen) to give a microbial strain, which was provided by Dr. Kuzushima (Aichi Cancer Center).

For the transformants for which expression was confirmed, single colonies on the culture plate were picked up and inoculated in 50 ml of the LB medium (containing 25 µg/ml kanamycin and 100 µg/ml ampicillin), and cultured for 16 hours. The cells were collected, and the medium was changed with the LB medium containing no antibiotics, to which glycerol was added to a final concentration of 20% and stored at -80°C (seed stock). The seed stock was inoculated to the LB medium containing antibiotics (25 µg/ml kanamycin and 100 µg/ml ampicillin) and cultured. After culturing, the antibiotics-containing medium was removed by centrifugation, changed with the antibiotics-free LB medium, and then mixed with an equal amount of 40% glycerol aqueous solution and stored at -80°C to prepare MCB.

### (2) Culturing

To 10 L of the antibiotics-free LB medium, 5 ml of MAGE-A4 MCB was inoculated. After preculturing at 37°C, 250 rpm, 1 vvm for 6-15 hours, 10 L of the preculture was inoculated to 200 L of the LB medium, and culturing was started at 30°C, 60 rpm, 1 vvm. When OD600 reached about 1, IPTG was added to a final concentration of 0.3 mmol/L to start induction, and DO control was maintained by agitation at about 60% (about 4.5 ppm) or more. Five hours after the start of the induction, culturing was terminated.

### (3) Cell collection and cell disruption

After the working cells were concentrated using Durapore PVDF GVPP 0.22 µm (V screen) 0.5 m² flat membrane (Millipore), an equal amount of 20 mmol/L Tris-HCl (pH 8.0) buffer was added three times or more to wash the cells, and 20 L of a cell slurry with a wet cell weight of 800 g was obtained. The cells were stored refrigerated or frozen.

To the thawed cells, 12 L of 20 mM Tirs-HCl (pH 8.0) was added and suspended to prepare a cell slurry, which was homogenized using a high pressure homogenizer at 10,000 psi until the slurry becomes transparent. A zeta potential filter, CUNO BioCap 2000 (60 M/120 M 2700 cm²) (Cuno) and a sterilization filter Sterashare (0.2 µm, 10 inch, 8600 cm²) (Cuno) were connected in series, and the disrupted liquid was filtered at ≤ 0.39 L/min (per set of the above filter) to remove the debris for clarification.

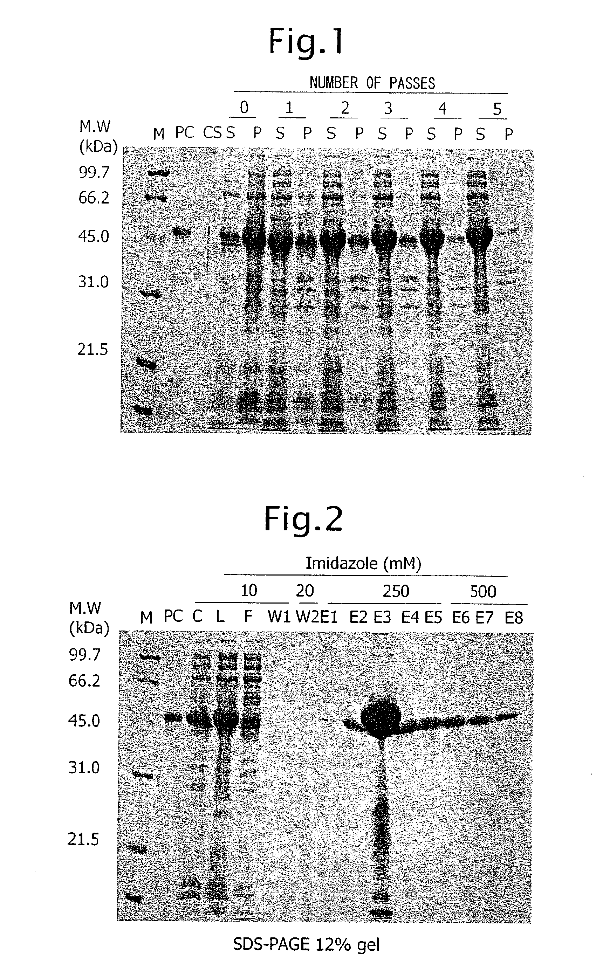

Fig. 1 shows the electrophoresis pattern of the supernatant of the disrupted liquid obtained.

Fig. 1 (number of passes and solubilization) shows that MAGE-A4 is present in the supernatant of the disrupted liquid of the cells obtained by culturing the MAGE-A4 recombinant Escherichia coli by the reduced SDS-PAGE of the disrupted liquid.

Symbols in the Fig. 1 are as follows: M: Molecular weight marker; PC: Positive control; CS: Culture supernatant; S: Supernatant; P: Precipitate.

### Production Example 2

### (Affinity column chromatography)

Using the Ni-NTA column (QIAGEN Ni-NT agarose, 25 cm in diameter, about 4 L), the MAGE-A4 protein was isolated from the disrupted filtrate. The step-wise elution is carried out as follows (flow rate 56 cm/h).

**[Table 1]**

| | |
|---|---|
| Equilibration | 20 mmol/L Tirs-HCl containing 300 mmol/L NaCl |
| | 20 mmol/L imidazole (pH 8.0) ≥5cv |
| Application | Disrupted liquid + 300 mmol/L NaCl + 20 mmol/L imidazole |
| Washing | 20 mmol/L Tirs-HCl containing 300 mmol/L NaCl |
| | 20 mmol/L imidazole (pH 8.0) ≥7cv |
| Elution | 20 mmol/L Tirs-HCl containing 300 mmol/L NaCl |
| | 100 mmol/L imidazole (pH 8.0) ≥5cv |

After starting elution, the A280 peak was sampled.

The electrophoresis pattern of the eluted liquid obtained is shown in Fig. 2.

Symbols in the Fig. 2 are as follows:
[Table 6]
   M: Marker
   PC: Positive control
   C: Culture liquid
   L: Disrupted supernatant

Fig. 2 shows that one-time purification yields MAGE-A4 with a purity of 95% or more by the reduced SDS-PAGE of the cell-disrupted liquid of His-Tag-MAGE-A4-expressing Escherichia coli and a fraction obtained by crude purification thereof with the Ni-NTA affinity column.

Using Millipore Pellicon 2 Ultracel PLCGC 10K 0.1 m² × one sheet of flat membrane (Millipore), the Ni-NTA column-eluted pooled liquid was concentrated to about 5 mg/mL. After concentration, the buffer was changed with 8 volume parts or more of 20 mM Tris-HCl (pH 8.0) to remove imidazole.

### Working Example 1

To the above concentrate, urea was added and dissolved to a final concentration of 8 M with the addition of 20mM Tris-HCl (pH 8.0), and then 60 mM tetrathionic acid dihydrate and 300 mM sodium sulfite at final concentrations were added sequentially, and stirred overnight at room temperature. The reaction mixture was passed through about 12 L of the Sephadex G-25 Coarse (GE Healthcare) 25 cm in diameter equilibrated with 10 mM sodium phosphate buffer containing 8 mol/L urea (pH 8.0) to exchange the buffer.

### Working Example 2

The reaction in Working Example 1 was also carried out in the presence of 6 M guanidine in stead of 8 M urea.

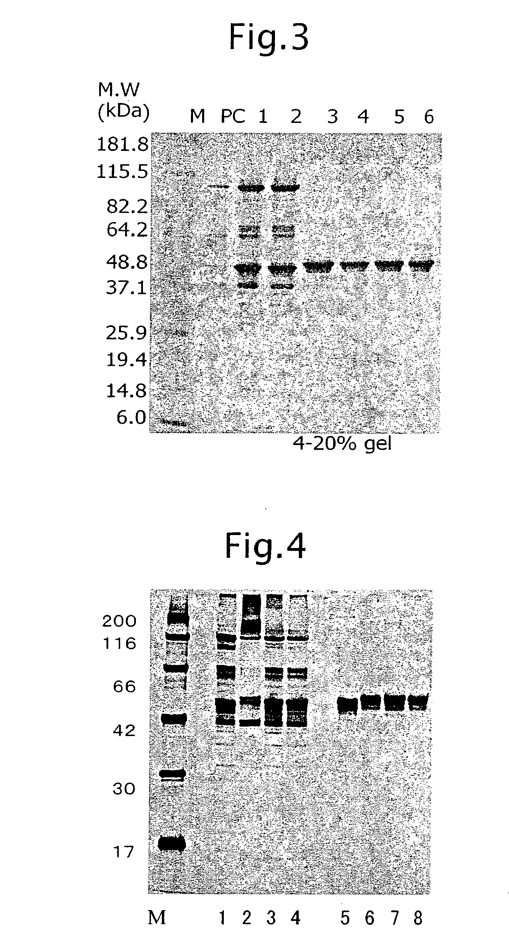

As shown in Fig. 3 (nonreduced SDS-PAGE of crude purified His-Tag MAGE-A4 and its sulfonation-protected product), convergence to one band (49 kd) was obtained in the case of 6 M guanidine as well as of 8 M urea.

Symbols in the Fig. 3 are as follows:
[Table 7]
   M: Marker
   PC: Control
   1: Before reaction (×1, 1.3 mg/mL)
   2: Before reaction (×4, 5.2 mg/mL)
   3: After reaction and purification (×1)
   4: After reaction and purification (×4) 6 M guanidine hydrochloride
   5: After reaction and purification (×1)
   6: After reaction and purification (×4) 8 M urea

### Working Example 3

Using the "crude purified His-Tag-MAGE-A4" obtained in Working Example 1, experiments in the following Working Examples 3 to 8 were carried out.

Fig. 3 shows that in the nonreduced SDS-PAGE of the crude purified His-Tag-MAGE-A4 and its sulfonation-protected product, the crude purified MAGE-A4 before the protection reaction is an assembly of multimers (60-110 kd) and structural isomers (38-48 kd), and that the sulfonation-protection reaction leads to convergence to one band (49 kd).

This is a molecular volume slightly larger than may be indicated from the band (45 kd) which is expected to be a SH product. This protected product exhibited a tendency to vary by H₂O₂ oxidation, but was shown to be stable to heat and freezing-thawing compared to the unprotected His-Tag MAGE-A4 (Fig. 4). Symbols in the Fig. 4 are as follows:
[Table 8]
   M: Marker
   1: MA4 0.3% H₂O₂-treated
   2: MA4 heat-treated
   3: MA4 freeze-thawed
   4: MA4 not treated

   5: Protected product 0.3% H₂O₂-treated
   6: Protected product heat-treated
   7: Protected product freeze-thawed
   8: Protected product not treated

Fig. 4 (nonreduced SDS-PAGE of the crude purified His-Tag-MAGE-A4 and its sulfonation-protedted product when treated under harsh conditions) shows that polymerized aggregation occurs before protection whereas the sulfonation-protected product is stable to heat and freezing-thawing.

### Working Example 4

### (Anion chromatography)

Further purification was carried out using the DEAE Sepharose Fast Flow, 13 cm in diameter, about 2L (GE Healthcare) equilibrated with 10 mM sodium phosphate buffer containing 8 mol/L urea (pH 8.0). The column procedure was gradient elution as described below. The flow rate was 25 cm/h for application and washing, and 75 cm/h for others.

**[Table 2]**

| | |
|---|---|
| Equilibration | 10 mM sodium phosphate buffer containing 8 mol/L urea (pH 8.0) ≥5cv |
| Application | Gel-filtrated pooled liquid |
| Washing | 10 mM sodium phosphate buffer containing 8 urea (pH 8.0) ≥5cv |
| Elution | A: 10 mM sodium phosphate buffer containing 8 mol/L urea (pH 8.0) |
| | B: 10 mM sodium phosphate buffer 8 mol/L urea and 1 mol/L NaCl (pH 8.0) |
| | 100% A: 0% B → 50% A: 50% B gradient, 20cv |

### Working Example 5

### (Hydroxyapatite column chromatography)

Using Pellicon 2 Ultracel PLCGC 10K, 0.1 m² × one sheet of flat membrane (Millipore), the DEAE-eluted pooled liquid was concentrated to about 3 mg/mL. After concentration, the buffer was changed with 6 volume parts or more of 1/2 D-PBS 8 mol/L urea for desalting. This was loaded to the D-PBS 8 mol/L urea-equilibrated HA ULTRAGEL, 10 cm in diameter, about 3 L (Pall) for further purification.

The condition for chromatography was gradient elusion described below. The flow rate was 25 cm/h from equilibration to elusion, and 40 cm/h for others.

**[Table 3]**

| | |
|---|---|
| Equilibration | 1/2 D-PBS 8 mol/L urea ≥5cv |
| Application | Concentration, desalting liquid |
| Washing | 1/2 D-PBS 8 mol/L urea ≥5cv |
| Elution | A: 1/2 D-PBS 8 mol/L urea |
| | B: 500 mM sodium phosphate buffer (pH 7.4) |
| | 100% A: 0% B → 0% A: 100% B, 10cv |

The impurities profile at each step of purification from the supernatant of the cell-disrupted liquid (total protein 57 g) is shown in the following table. It was demonstrated that endotoxins, host-derived proteins, DNA etc. have efficiently been removed from the Ni-NTA crude-purified protein.

**[Table 4]**

| Step | Protein | Endotoxin | Host protein | DNA |
|---|---|---|---|---|
| | g | EU/mL | ng/mL | pg/mL |
| Supernatant of cell-disrupted liquid | 57.0 | | | |
| Ni-affinity column-eluted liquid | 7.3 | 16,200 | 625 | <1,000,000 |
| DEAE column-eluted liquid | 5.9 | 43,300 | 0.2 | 239,000 |
| Hydroxyapatite column-eluted liquid | 4.5 | 2,300 | 0.1 | <100,000 |
| DEAE column-eluted liquid | 2.6 | 31 | 0.1 | <200 |

### Working Example 6

### (CHP embedding)

To the thus obtained sulfonated His-Tag-MAGE-A4, 12 weight parts of CHP 60T or 100T (manufactured by NOF Corp.) was mixed in the presence of 6 M urea or 6 M urea + 2-mercaptoethanol (2ME). After this was diluted once in the buffer, concentration and dilution by ultrafiltration were repeated to remove the excess reagents together with the buffer exchange. After the bulk liquid obtained was sterilization-filtered, it was aliquoted into vials to prepare a vaccine formulation.

In the "SE-HPLC chart immediately after the mixing of CHP and protein" obtained, the protein peak that otherwise appears at 9.7 minutes by UV (280 nm) detection was not observed, and moved to 6.5 minutes in agreement with the CHP peak. The peak at 14.1 minutes was that of urea.

On the other hand, in the "SE-HPLC chart" after buffer exchange (removal of urea) by dilution and concentration, the urea peak at 14.1 minutes disappeared and the peak of CHP-His-Tag-MAGE-A4 at 6.5 minutes was recognized.

### Working Example 7

### (Confirmation of vaccine effect)

The immunological activity of the CHP-His-Tag-MAGE-A4 vaccine thus obtained was confirmed using the ELISPOT method as described below.

On day 7, peripheral blood was taken from HLA-A24-positive healthy donors, and using magnet beads (Miltenyi) coated with anti-CD14 antibody, CD14-positive mononuclear cells were separated, and cultured in the presence of GM-CSF and IL-4. These CD14-positive mononuclear cells which differentiate into dendritic cells during culturing were recovered on day 1, and suspended in the culture medium to a concentration of 1 × 10⁶ cells/ml.

To the dendritic cells, CHP-His-Tag-MAGE-A4 (7.5-11 µg protein/mL), CHP-His-Tag-Her2 (20 µg protein/mL), or MAGE-A4-derived HLA-A24-restrictive antigen peptide (p143, 10 µg/mL) was added, and incubated at 37°C, 5% CO2 for 3 hours.

After washing these cells with a fresh medium, they were plated at 5 × 10⁴ cells/well to the ELISPOT plate (Millipore) that had previously coated with anti-human IFN-γ antibody and had been blocked with a 10% fetal bovine serum-containing medium. Various concentrations of MAGE-A4-CTL clone 2-28 (see Miyahara Y. et al., Clin. Cancer Res., 2005, 11(5): 5581-9) were added, and cultured at 37°C, 5% CO2.

Twenty-four hours later, they were washed in a phosphate buffered saline (PBS) containing 0.01% Tween 20, to which a biotinylated anti-human IFN-γ antibody was added and incubated overnight at 4°C. After washing in 0.01% Tween 20-containing PBS, streptavidin-labelled alkaline phosphatase was added and reacted at room temperature for 1.5 hour. After futher washing in 0.01% Tween 20-containing PBS, a chromogenic substrate (BioRad) for alkaline phosphatase was added, and color development reaction was carried out for several minutes. The plate was washed in distilled water to stop the development reaction, and the IFN-γ-specific spots obtained were counted.

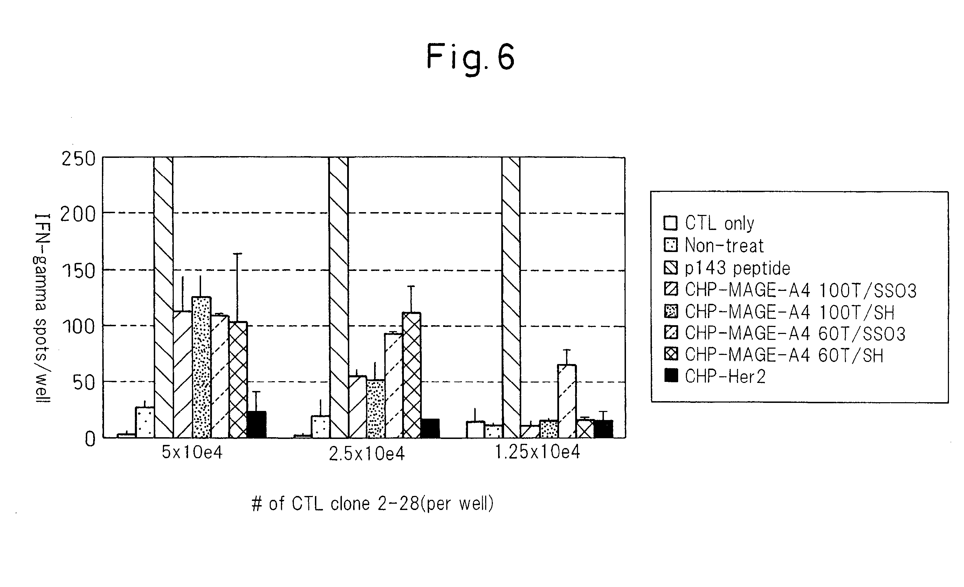

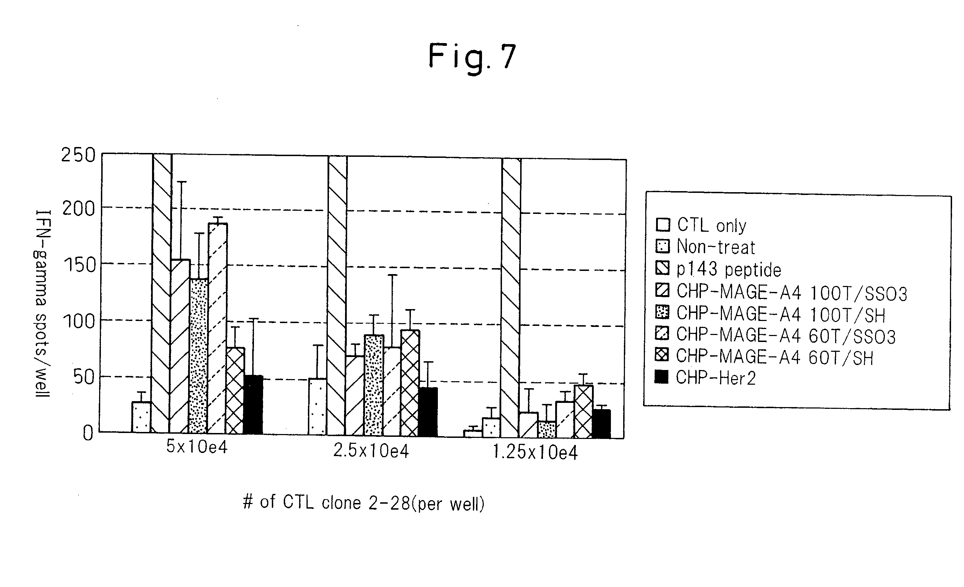

The results obtained are shown in Fig. 6 and Fig. 7. In Fig. 6, the conditions etc. are as follows:
[Table 9]
   Evaluation of the Activity of CHP-MAGE-A4 ELISPOT assay, Dec-29, 2005
   (Immature DC from Wang-san was used as APC)

In Fig. 7, the conditions etc. are as follows:
[Table 10]
   Evaluation of the Activity of CHP-MAGE-A4 ELISPOT assay, Dec-29, 2005
   (Immature DC from Okumura-san was used as APC)

Figs. 6 and 7 revealed that in the CHP-His-Tag-MAGE-A4-administration group, more IFN-γ spots were recognized compared to the negative control (Non-treat or CHP-His-Tag-Her2-administration group), confirming that CHP-His-Tag-MAGE-A4 was incorporated by dendritic cells and was activating CTL.

### Working Example 8 (Measurement of size distribution)

The size distribution of the embedded product was measured. The condition for measurement is as described in the following table 5.

**[Table 5]**

| Sample Details | | | | | |
|---|---|---|---|---|---|
| Sample Name: | CHP-MAGE-A4 | | | | |
| SOP Name: | Manual measurement settings | | | | |
| General Notes: | Lot.No.IS01, non filtered | | | | |
| | | | | | |
| File Name: | sawada.dts | Dispersant Name: | | PBS | |
| Record Number: | 45 | Dispersant RI: | | 1.335 | |
| Material RI: | 1.45 | Viscosity(cP): | | 1.0200 | |
| Material Absorbtion: | 0.00 | Measurement Date and Time: | | January 17, 2006 19:54:13 | |

| System | | | | | |
|---|---|---|---|---|---|
| Temperature (°C): | 25.0 | Duration Used (s): | | 60 | |
| Count Rate (kcps.) : | 354.1 | Measurement Position (mm) : | | 4.20 | |
| Cell Description: | Low volume glass cuvette (4... | | Attenuator: | 7 | |

| Results | | | | | |
|---|---|---|---|---|---|
| | | | Diam. (nm) | %Intensity | Width (nm) |
| Z-Average (d.nm) : | 48.4 | Peak1 : | 72.9 | 100.0 | 52.7 |
| Pdl: | 0.271 | Peak2 : | 0.00 | 0.0 | 0.00 |
| Intercept: | 0.951 | Peak3: | 0.00 | 0.0 | 0.00 |

The result of measurement is shown in the graph in Fig. 8 (Size Distribution by Intensity).

## Claims

1. A process for producing a composition comprising a cancer-testis antigen-hydrophobized polysaccharide complex, which comprises a cancer-testis antigen having two or more cysteine residues in a molecule thereof, and a hydrophobized polysaccharide; the process comprising:
(a) sulfonating the cysteine residues of the cancer-testis antigen;
(b) purifying the sulfonated cancer-testis antigen;
(c) embedding the purified cancer-testis antigen into the hydrophobized polysaccharide, to thereby provide the cancer-testis antigen-hydrophobized polysaccharide complex; and
(d) reducing the complex.

2. The process for producing a composition comprising a cancer-testis antigen-hydrophobized polysaccharide complex according to claim 1, wherein the sulfonation is reversible.

3. The process for producing a composition comprising a cancer-testis antigen-hydrophobized polysaccharide complex according to claim 1, wherein the cancer-testis antigen (CTA) is a recombinant testis antigen expressed using a recombinant Eschericha coli.

4. The process for producing a composition comprising a cancer-testis antigen-hydrophobized polysaccharide complex according to claim 1, wherein the sulfonation is carried out by allowing a thiosulfate and a tetrathionate to act on the protein to sulfonate the thiol groups of the protein in order to protect them.

5. The process for producing a composition comprising a cancer-testis antigen-hydrophobized polysaccharide complex according to claim 1, wherein the purification is carried out by chromatography.

6. The process for producing a composition comprising a cancer-testis antigen-hydrophobized polysaccharidecomplex according to claim 1, wherein the cancer-testis antigen is one or a plurality of CTA's selected from the melanoma associated antigen/melanoma antigen gene (MAGE) family of MAGE A1, MAGE A2, MAGE A3, MAGE A4, MAGE A5, MAGE A6, MAGE A7, MAGE A8, MAGE A9, MAGE A10, MAGE A11, MAGE A12, MAGE B1, MAGE B2, MAGE B3, MAGE B4, MAGE C1 and MAGE C2.

7. The process for producing a composition comprising a cancer-testis antigen-hydrophobized polysaccharide complex according to claim 1, wherein the recombinant testis antigen is one or a plurality of CTA's selected from NY-ESO-1, the LAGE family, the GAGE family, the SAGE family, and the XAGE family.

8. The process for producing a composition comprising a cancer-testis antigen-hydrophobized polysaccharide complex according to claim 1, wherein the recombinant testis antigen is His-Tag-added His-Tag-CTA.

## Patentansprüche

1. Verfahren zur Herstellung einer Zubereitung, umfassend einen Komplex aus einem Hodenkrebsantigen und einem hydrophobisierten Polysaccharid, der ein Hodenkrebsantigen umfasst, enthaltend zwei oder mehr Cysteinreste in einem Molekül davon, und ein hydrophobisiertes Polysaccharid, wobei das Verfahren die
(a) Sulfonierung des Cysteinrestes des Hodenkrebsantigens,
(b) Reinigung des sulfonierten Hodenkrebsantigens,
(c) Einbettung des gereinigten Hodenkrebsantigens in das hydrophobisierte Polysaccharid, wodurch ein Komplex aus Hodenkrebsantigen und hydrophobisiertem Polysaccharid erhalten wird,
(d) Reduktion des Komplexes
umfasst.

2. Das Verfahren zur Herstellung einer Zubereitung, umfassend einen Komplex aus einem Hodenkrebsantigen und einem hydrophobisierten Polysaccharid nach Anspruch 1, worin die Sulfonierung reversibel ist.

3. Das Verfahren zur Herstellung einer Zubereitung, umfassend einen Komplex aus einem Hodenkrebsantigen und einem hydrophobisierten Polysaccharid nach Anspruch 1, worin das Hodenkrebsantigen (CTA) ein rekombinantes Hodenantigen ist, exprimiert mittels eines rekombinanten Eschericha coli.

4. Das Verfahren zur Herstellung einer Zubereitung, umfassend einen Komplex aus einem Hodenkrebsantigen und einem hydrophobisierten Polysaccharid, worin die Sulfonierung so durchgeführt wird, dass es einem Thiosulfat und einem Tetrathionat erlaubt in der Art auf das Protein einzuwirken, dass die Thiolgruppen des Proteins sulfoniert werden, um diese zu schützen.

5. Das Verfahren zur Herstellung einer Zubereitung, umfassend einen Komplex aus einem Hodenkrebsantigen und einem hydrophobisierten Polysaccharid nach Anspruch 1, worin die Reinigung mittels Chromatographie erfolgt.

6. Das Verfahren zur Herstellung einer Zubereitung, umfassend einen Komplex aus einem Hodenkrebsantigen und einem hydrophobisierten Polysaccharid nach Anspruch 1, worin das Hodenkrebsantigen eines oder eine Vielfalt von CTAs ist, ausgewählt aus der Melanom-assoziierten-Antigen/Melanomantigen Gen (MAGE) Familie der MAGE A1, MAGE A2, MAGE A3, MAGE A4, MAGE A5, MAGE A6, MAGE A7, MAGE A8, MAGE A9, MAGE A10, MAGE Al 1, MAGE Al2, MAGE B1, MAGE B2, MAGE B3, MAGE B4, MAGE Cl und MAGE C2.

7. Das Verfahren zur Herstellung einer Zubereitung, umfassend einen Komplex aus einem Hodenkrebsantigen und einem hydrophobisierten Polysaccharid nach Anspruch 1, worin das rekombinante Hodenkrebsantigen eines oder eine Vielfalt von CTAs ist, ausgewählt aus NY-ESO-1, der LAGE-Familie, der GAGE-Familie, der SAGE-Familie und der XAGE-Familie.

8. Das Verfahren zur Herstellung einer Zubereitung, umfassend einen Komplex aus einem Hodenkrebsantigen und einem hydrophobisierten Polysaccharid nach Anspruch 1, worin das rekombinante Hodenkrebsantigen His-Tag-added His-Tag-CTA ist.

## Revendications

1. Procédé de production d'une composition comprenant un complexe antigène testiculaire du cancer-polysaccharide rendu hydrophobe, qui comprend un antigène testiculaire du cancer ayant deux ou plus résidus cystéine dans une molécule de celui-ci, et un polysaccharide rendu hydrophobe ; le procédé comprenant :
(a) la sulfonation des résidus cystéine de l'antigène testiculaire du cancer ;
(b) la purification de l'antigène testiculaire du cancer sulfoné ;
(c) l'incorporation de l'antigène testiculaire du cancer purifié dans le polysaccharide rendu hydrophobe, afin d'ainsi obtenir le complexe antigène testiculaire du cancer-polysaccharide rendu hydrophobe ; et
(d) la réduction du complexe.

2. Procédé de production d'une composition comprenant un complexe antigène testiculaire du cancer-polysaccharide rendu hydrophobe selon la revendication 1, dans lequel la sulfonation est réversible.

3. Procédé de production d'une composition comprenant un complexe antigène testiculaire du cancer-polysaccharide rendu hydrophobe selon la revendication 1, dans lequel l'antigène testiculaire du cancer (CTA) est un antigène testiculaire par recombinaison exprimé en utilisant une Escherichia coli par recombinaison.

4. Procédé de production d'une composition comprenant un complexe antigène testiculaire du cancer-polysaccharide rendu hydrophobe selon la revendication 1, dans lequel la sulfonation est réalisée en permettant à un thiosulfate et à un tétrathionate d'agir sur la protéine pour obtenir une sulfonation des groupes thiols de la protéine afin de les protéger.

5. Procédé de production d'une composition comprenant un complexe antigène testiculaire du cancer-polysaccharide rendu hydrophobe selon la revendication 1, dans lequel la purification est réalisée par chromatographie.

6. Procédé de production d'une composition comprenant un complexe antigène testiculaire du cancer-polysaccharide rendu hydrophobe selon la revendication 1, dans lequel l'antigène testiculaire du cancer est un ou une pluralité de CTA choisi(e) parmi la famille d'antigènes associés au mélanome/gènes d'antigène de mélanome (MAGE) MAGE A1, MAGE A2, MAGE A3, MAGE A4, MAGE A5, MAGE A6, MAGE A7, MAGE A8, MAGE A9, MAGE A10, MAGE A11, MAGE A12, MAGE B1, MAGE B2, MAGE B3, MAGE B4, MAGE C1 et MAGE C2.

7. Procédé de production d'une composition comprenant un complexe antigène testiculaire du cancer-polysaccharide rendu hydrophobe selon la revendication 1, dans lequel l'antigène testiculaire par recombinaison est un ou une pluralité de CTA choisi(e) parmi NY-ESO-1, la famille LAGE, la famille GAGE, la famille SAGE et la famille XAGE.

8. Procédé de production d'une composition comprenant un complexe antigène testiculaire du cancer-polysaccharide rendu hydrophobe selon la revendication 1, dans lequel l'antigène testiculaire par recombinaison est un CTA-marqueur His auquel a été ajouté un marqueur His.


### SEQUENCE LISTING

<110> ImmunoFrontier, Inc.
<120> Kenji, Nagura; Naozumi, Harada; Masahiro, Urushibara
<130> ImmunoFrontier-1063298
<140> 2006-108668
   <141> 2006-04-11
<160> 2
<170> PatentIn version 3.3
<210> 1
   <211> 27
   <212> DNA
   <213> Forward primer
<400> 1
   ggatccatgt cttctgagca gaagagt 27
<210> 2
   <211> 25
   <212> DNA
   <213> Reverse primer
<400> 2
   aagcttctca tgctcagact ccctc 25